# EUROPEAN PATENT APPLICATION

(11) **EP 3 260 126 A1**
(43) Date of publication of application: **27.12.2017**
(21) Application number: 17181229.0
(22) Date of filing: 08.10.2010
(51) Int. Cl.: A61K 35/744, A61K 35/66, A61K 39/02, A61K 9/48, A61P 25/00, A23P 10/30, A23L 33/10, A23L 33/135

(54) **COMPOSITIONS AND METHODS COMPRISING PEDIOCOCCUS FOR REDUCING AT LEAST ONE SYMPTOM ASSOCIATED WITH AUTISM SPECTRUM DISEASE IN A PERSON DIAGNOSED WITH AN AUTISM SPECTRUM DISEASE**

(30) Priority: 09.10.2009 US 250220 P
(62) Divisional of application: 10822796.8
(71) Applicant: Prothera, Inc., Reno, Nevada 89521 (US)
(72) Inventor: OLMSTEAD, Stephen F., Reno, Nevada 89519 (US)
(74) Representative: Schüssler, Andrea

(57) **Abstract**

The inventions concerns compositions comprising at least one Pediococcus strain and at least one other probiotic microbial organism that reduce one or more symptoms of an autism spectrum disorder such as autism. The compositions can be used, for example, as dietary supplements, food additives, and pharmaceutical preparations.

## Description

### CROSS-REFERENCE TO RELATED APPLICATIONS

The present application claims the benefit of copending United States Provisional Patent Application Serial No. 61/250,220, filed October 9, 2009, which application is incorporated herein by reference in its entirety.

### INCORPORATION BY REFERENCE

The present application incorporates by reference the following references, publications and patents in their entirety to the extent they assist in the general understanding of the novel subject matter discussed herein, but not for limitations within the claims:

**U.S. Patent Documents**

| **US Patent** | **Date** | **Inventor** |
|---|---|---|
| 5,445,835 | August, 1995 | Vedamuthu |
| 5,716,615 | February, 1998 | Cavaliere |
| 6,241,983 | Jun 5, 2001 | Paul et al. |
| 6,251,391 | June 26, 2001 | Wilkinson |
| 6,294,166 | September, 2001 | Hsia |
| 6,447,772 | September, 2002 | Houston |
| 6,468,525 | October, 2002 | Watson et al. |
| 6,783,780 | August, 2004 | De Jong et al. |
| 6,899,876 | May, 2005 | Houston |
| 7,241,441 | July 10, 2007 | Choi |
| 7,307,062 | December, 2007 | Bolte |
| | | |

| **US Patent Application** | **Publication Date** | **Inventor** |
|---|---|---|
| 2004/0005304 | January 8, 2004 | Brudnak |
| 2004/0062757 | April 1, 2004 | Finegold |
| 2004/0170617 | September 2,2004 | Finegold |
| 2005/0186188 | August 25,2005 | Guo |
| 2006/0008511 | January 12, 2006 | Lin et al. |
| 2006/0165661 | July 27, 2006 | Speelmans |
| 2006/0233777 | October 19,2006 | Piva |
| 2006/0251633 | November 9, 2006 | Salvadori et al. |
| 2007/0098705 | May 3, 2007 | Ljungh-Wadstrom |
| 2007/0280911 | December 6, 2007 | Cobb et al. |
| 2007/0286916 | December 13, 2007 | Bengmark |
| 2007/0160589 | July 12,2007 | Mattson |
| 2007/0122397 | May 31, 2007 | Sanguansri et al. |
| | | |

| | **World Patent Documents** | |
|---|---|---|
| WO 2004/110466 | December 23, 2004 | Speelmans et al. |
| WO 01/93904 | December 13, 2001 | Finegold |

### Other References

Ashwood P, Wills S, Van de Water J. The immune response in autism: a new frontier for autism research. J Leukoc Biol 2006;80:1-15.
Axelsson, L. Lactic acid bacteria: classification and physiology. In, Lactic Acid Bacteria: Microbiology and Functional Aspects, 1998; 2nd ed; S. Salminen and A. Von Wright (eds); pp. 1-72. Marcel Dekker, Inc, New York.
Barros RR, Carvalho MD, Peralta JM, Facklam RR, Teixeira LM. Phenotypic and genotypic characterization of Pediococcus strains isolated from human clinical sources. J Clin Microbiol 2001:39:1241-6.
Beresford TP, Fitzsimons NA, Brennan NL, Cogan TM. Recent advances in cheese microbiology. Int. Dairy J. 2001;11:259-74.
Bhunia AK, Johnson MC, Ray B. Purification, characterization and antimicrobial spectrum of a bacteriocin produced by Pediococcus acidilactici. J Appl Bacteriol 1988;65:261-8.
Black F, Einarsson K, Lidbeck A, Orrhage K, Nord CE. Effect of lactic acid producing bacteria on the human intestinal microflora during ampicillin treatment. Scand J Infect Dis 1991;23:247-54
Black C, Kaye J, Jick H. Relation of childhood GI disorders to autism: Nested case-control study using data from the UK General Practice Research Database. Br. Med. J. 2002; 325: 429-421.
Bolte ER. Autism and clostridium tetani. Med Hypotheses 1998;51:133-44.
Brudnak MA, Rimland B, Terry RE, et al. Enzyme-based therapy for autism spectrum disorders is it worth another look? Med. Hypotheses 2002;58:422-8.
Campbell DB, et al. A genetic variant that disrupts MET transcription is associated with autism. Proc Nat Acad Sci 2006;103:16834-9.
Erickson CA, Stigler KA, Corkins MR, Posey DJ, Fitzgerald JF, McDougle CJ. Gastrointestinal factors in autistic disorder: a critical review. J Autism Dev Divord 2006;35:713-27.
Daeschel MA, Klaenhammer TR. Association of a 13.6-megadalton plasmid in Pediococcus pentosaceus with bacteriocin activity. Appl Environ Microbiol 1985;50:1528-41.
Dambekodi PC, Gilliland SE. Incorporation of cholesterol into the cellular membrane of Bifidobacterium longum. J Dairy Sci 1998; 81:1818-1824.
D'Eufemia P, Celli M, Finocchiaro R, et al. Abnormal intestinal permeability in children with autism. Acta Paediatr 1996; 85:1076-9.
Drider D, Fimland G, Hechard Y, McMullen LM, Prévost H. The continuing story of class IIa bacteriocins. Microbiol Mol Biol Rev 2006; 70:564-82
Dunne C, O'Mahoney L, Murphy L, et al. In vitro selection criteria for probiotic bacteria of human origin: correlation with in vivo findings. Am J Clin Nutr 2001;73(2 Suppl): 3865-925.
Garvie EI. Genus Pediococcus. In, Bergey's Manual of Systematic Bacteriology, vol 2; 9th ed; pp. 1075-9. Sneath PH, Mair NS, Sharpe ME, Holt JG (eds.); 1986;Williams and Wilkins, Baltimore.
Elmer GW, McFarland LV. Suppression by Saccharomyces boulardii of toxigenic Clostridium difficile overgrowth after vancomycin treatment in hamsters. Antimicrob Agents Chemother 1987;31:129-31.
Fimland G, Johnsen L, Dalhus B, Nissen-Meyer J. Pediocin-like antimicrobial peptides (class IIa bacteriocins) and their immunity proteins: biosynthesis, structure, and mode of action. J Pept Sci 2005;11:688-96.
Gonzalez CF, Kunka BS. Evidence for plasmid linkage of raffinose utilization and associated alpha-galactosidase and sucrose hydrolase activity in Pediococcus pentosaceus. Appl Environ Microbio 1986; 51.105-9.
Graham DC, McKay LL. Plasmid DNA in strains of Pediococcus cerevisiae and Pediococcus pentosaceus. Appl Environ Microbiol 1985;50:532-4*.*
Guslandi M, et al. Saccharomyces boulardii in maintenance treatment of Crohn's disease. Dig Dis Sci 2000;45:1462-4.
Holzapfel WH, Haberer P, Geisen R, Björkroth J, Schillinger U. Taxonomy and important features of probiotic microorganisms in food and nutrition. Am J Clin Nutr 2001;73(2 Suppl) 365S-73S.
Horvath K, Perman JA. Autism and gastrointestinal symptoms. Curr Gastroenterol Rep 2002;4:251-8.
Horvath K, Perman JA. Autistic disorder and gastrointestinal disease. Curr Opin Pediatr 2002; 14:583-7.
Ishibashi N, Yamazaki S. Probiotics and safety. Am J Clin Nutr 2001;73(suppl):465S-70S.
Jack RW, Tagg JR, RayB. Bacteriocins of Gram positive bacteria. Microbiol. Rev. 1995; 59: 171-200.
Jack RW, Bierbaum G, Sahl H-G. Lantibiotics and Related Peptides. 1998; Berlin: Springer-Verlag.
Jiang TA, Mustapha A, Savaiano DA. 1996. Improvement of lactose digestion in humans by ingestion of unfermented milk containing Bifidobacterium longum. J. Dairy Sci 1996;79:750-7.
Jyonouchi H, Geng L, Ruby A, Zimmerman-Bier B. Dysregulated innate immune responses in young children with autism spectrum disorders: their relationship to gastrointestinal symptoms and dietary intervention. Neuropsychobiology 2005;51:77-85. Klaenhammer TR. Genetics of bacteriocins produced by lactic acid bacteria. FEMS Microbial. Rev. 1993; 12, 39-85.
Klare I, Konstabel C, Werner G, et al. Antimicrobial susceptibilities of Lactobacillus, Pediococcus and Lactococcus human isolates and cultures intended for probiotic or nutritional use. J Antimicrob Chemother 2007;59:900-12.
Konings RNH, Hilbers CW. Lantibiotics: a unique group of antibiotic peptides. Antonie Leeuwenhoek (Special issue) 1996;69:87-202.
Kruszewska K, Lan J, Lorca G, Yanagisawa N, Marklinder I, Ljungh Å. Selection of lactic acid bacteria as probiotic strains by in vitro tests. Microecol Ther 2002; 29:37-49.
Lee S, Lillehoj HS, Park DW, Hong YH, Lin JJ. Effects of Pediococcus- and Saccharomyces-based probiotic (MitoMax®) on coccidiosis in broiler chickens. Comp. Immunol., microbial. Infect. Dis. 2007;30:261-8.
Levy SE, Hyman SL. Novel treatments for autistic spectrum disorders. Ment. Retard. Dev. D.R. 2005;11:131-42
Ljungh Å, Lan J, Yanagisawa N. Isolation, selection and characteristics of Lactobacillus paracasei ssp paracasei F19. Microbial Ecol Health Dis 2002;14 Suppl 3:4-6.
MacFarlane S, MacFarlane GT, Cummings JH. Review article: prebiotics in the gastrointestinal tract. Aliment Pharmacol Ther 2006;24:701-14.
Marugg JD, Gonzalez CF, Kunka BS, et al. Cloning, expression, and nucleotide sequence of genes involved in production of pediocin PA-1, a bacteriocin from Pediococcus acidilactici PAC1.0. Appl Environ Microbiol 1992;58:2360-7.
Mathys S, von Ah U, Lacroix C, et al. Detection of the pediocin gene pedA in strains from human faeces by real-time PCR and characterization of Pediococcus acidilactici UVA1. BMC Biotechnol 2007;7:55*.*
Millette M, Dupont C, Archambault D, Lacroix M. Partial characterization of bacteriocins produced by human Lactococcus lactis and Pediococccus acidilactici isolates. J Appl Microbiol 2007;102:274-282.
Mora D, Fortina MG, Parini C, Daffonchio D, Manachini PL. Genomic subpopulations within the species Pediococcus acidilactici detected by multilocus typing analysis: relationships between pediocin AcH/PA-1 producing and non-producing strains. Microbiol. 2000;146:2027-38.
Motlagh, A, Bukhtiyarova M, Ray B. Complete nucleotide sequence of pSMB74, a plasmid encoding the production of pediocin AcH in Pediococcus acidilactici. Lett Appl Microbiol 1994;18:305-12.
Nes IF, Diep DB, Håvarstein IS, Brurberg MB, Eijsink V, Holo H. Biosynthesis of bacteriocins in lactic acid bacteria. Antonie van Leeuwenhoek 1996;70:113-128.
New York State Department of Health Early Intervention Program. (1999). Clinical Practice Guideline: Report of the Recommendations. AutismlPervasive Developmental Disorders, Assessment and Intervention for Young Children (Age 0-3 Years) (Publication No. 4215, pp. 163-194). Albany, NY.
Okereke A, Montville TJ. Bacteriocin inhibition of Clostridium botulinum spores by lactic acid bacteria. J Food Prot 1991;1,349-53.
O'Sullivan DJ. Screening of intestinal microflora for effective probiotic bacteria. J Ag Food Chem 2001;49:1751-60.
Parracho HM, Bingham MO, Gibson GR, McCartney AL. Differences between the gut microflora of children with autistic spectrum disorders and that of healthy children. J Med Microbiol 54:987-91.
Parvez S, Malik KA, Ah Kang S, Kim HY. Probiotics and their fermented food products are beneficial for health. *J Appl Microbiol* 2006*;***100**:1171-85*.*
Pucci MJ, Vedamuthu ER, Kunka BS, Vandenbergh PA. Inhibition of Listeria monocytogenes by using bacteriocin PA-1 produced by Pediococcus acidilactici PAC1.0. *Appl Environ Microbiol* 1988*;***54**:2349-53*.*
Reddy BS, Rivenson A. Inhibitory effect of Bifidobacterium longum on colon, mammary, and liver carcinogenesis induced by 2-amino-3-methylimidazo[4,5-f] quinoline, a food mutagen. Cancer Res 1993; 53:3914-8.
Riebel WJ, Washington JA. Clinical and Microbiologic Characteristics of Pediococci. J Clin Microbiol 1990;28:1348-55
Rodriguez JM, Martinez MI, Kok J. Pediocin PA-1, a wide-spectrum bacteriocin from lactic acid bacteria. Crit Rev Food Sci Nutr 2002; 42:91-121.
Rolfe RD. The role of probiotic cultures in the control of gastrointestinal health. J Nutr 2000; 130:396S-402S.
Sahl HG, Jack RW, Bierbaum G. Biosynthesis and biological activities of lantibiotics with unique post-translational modifications. Eur. J. Biochem. 1995;230:827-53. Salminen S. Human studies on probiotics: aspects of scientific documentation. Scand J Nutr 2001;45:8-12.
Sandler RH, Finegold SM, Bolte ER, et al. Short-term benefit from oral vancomycin treatment of regressive-onset autism. JCN 2000;15:429-35.
Simpson WJ, Taguchi H. The genus Pediococcus, with notes on the genera Tetratogenococcus and Aerococcus, In Wood BJB, Holzapfel WH (eds). The Genera of Lactic Acid Bacteria. 1995; pp. 125-172; Chapman & Hall, London.
Surawicz CM, McFarland LV, Greenberg RN, et al. The search for a better treatment for recurrent Clostridium difficile disease: use of high-dose vancomycin combined with Saccharomyces boulardii. Clin Infect Dis 2000;31:1012-7.
Surawicz CM, McFarland LV, Elmer G, Chinn J. Treatment of recurrent Clostridium difficile colitis with vancomycin and Saccharomyces boulardii. Am J Gastroenterol 1989;84:1285-7.
Takahashi T, Nakagawa E, Nara T, Yajima T, Kuwata T. Effects of orally ingested Bifidobacterium longum on the mucosal IgA response of mice to dietary antigens. Biosci Biotechnol Biochem 1998;62:10-5
Tannock GW. Identification of Lactobacilli and Bifidobacteria. Current Issues Molec. Biol 1999;1:53-64.
Tissier H. Traitement des infections intestinales per la methode de la flora bacterienne de l'intestin. C R Soc. Biol. 1906;60:359-61.
Valicenti-McDermott M, McVicar K, Rapin I, Wershil BK, Cohen H, Shinnar S. Frequency of gastrointestional symptoms in children with autistic spectrum disorders and association with family history of autoimmune disease. J Dev Behav Pediatr 2006;27(2 Suppl):S128-36.
Walter J, Hertel C, Tannock GW, Lis CM, Munro K, Hammes WP. Detection of Lactobacillus, Pediococcus, Leuconostoc, and Weissella species in human feces by using group-specific PCR primers and denaturing gradient gel electrophoresis. Appl Environ Microbiol 2001;67:2578-85*.*
White JF. Intestinal pathophysiology in autism. Exp Biol Med (Maywood) 2003;228:639-49.
Whorwell PJ, Altringer L, Morel J, et al. Efficacy of an encapsulated probiotic Bifidobacterium infantis 35624 in women with irritable bowel syndrome. Am J Gastroenterol 2006;101:1581-90.
Zareie M, Johnson-Henry K, Jury J, et al. Probiotics prevent bacterial translocation and improve intestinal barrier function in rats following chronic psychological stress. Gut 2006;55:1553-60.

### BACKGROUND

Autism spectrum disorders (ASDs) are generally regarded as diseases comprising abnormalities in brain structure and/or function. ASDs appear in early childhood and are characterized by core symptoms of impaired social relatedness, delayed language, and restricted patterns of behavior. In addition to core symptoms, children with autism frequently exhibit serious behavioral disturbances, such as self-injury, aggression, hyperactivity, and tantrums in response to routine environmental demands and stimuli. ASDs are also associated with an array of gastrointestinal, immune system, and metabolic abnormalities (Ashwood et al., 2006; D'Eufemia et al., 1996; Erickson et al., 2006; Horvath & Perman, 2002; Jyonouchi et al., 2005; Valicenti-McDermott, 2006; White, 2003).

ASDs affect as many as 1 child per 150, and estimates show that there could be up to 1.5 million autistic people in the United States today. Therefore, effective dietary and/or pharmaceutical interventions for ASD could have a major public health impact.

Thus, there has gone unmet a need for improved methods, compositions, etc., that reduce one or more symptoms associated with autism. The present systems and methods, etc., provide these and/or other advantages.

### SUMMARY

In one aspect, the compositions, methods, systems, etc., herein are directed to providing probiotic compositions that are capable of reducing one or more, typically two or more, and more typically three or more symptoms of ASD in individuals having ASD. The compositions, formulations, methods, etc., can be used as dietary supplements or as food additives or as pharmaceutical agents or otherwise as desired to reduce symptoms of ASD. The methods, etc., herein include methods, kits, labels, systems, etc., directed to labeling, marketing and otherwise providing the compositions to health care professionals and/or to consumers for use in reducing symptoms of Autism Spectrum Disorders.

The compositions may be used as dietary supplements, food and beverage additives, and as pharmaceutical agents for reducing the symptoms of autism in a human in need thereof.

It is not necessary that individuals with autism have an antecedent use of antibiotics, or that they have been diagnosed with a microbial infection or bacterial overgrowth, in order to benefit from the compositions, methods, systems, etc., herein.

The inclusion of at least one strain of *Pediococcus* in the composition is essential for this invention.

In one embodiment, the composition is comprised of a mixture of a first microbial organism comprising at least one strain of *Pediococcus* and at least one second microbial organism having a beneficial health effect in humans, wherein the second microbial organism is at least one of a bacterium or a fungus. Typically, at least one additional microbial organism is selected from the group comprising Lactobacillus, Bifidobacterium or Saccharomyces.

In a further embodiment, the compositions, etc., are provided in capsules or other suitable administration formats, and a single capsule provides a full serving or dose. Generally speaking, a serving is an individual, full quantity of food or drink. Nutritional supplements and the like are typically considered foods, and thus herein the term "serving" is the term used for a full portion of supplement, which can be, for example, 1 capsule, 1/4 teaspoon, or 6 tablets. Dose is a full quantity of medication to be taken at one time. As used herein, both indicate a full portion to be taken by or administered to a recipient at a single time.

In one example, each serving or dose comprises at least about 1 million and up to 150 billion Colony Forming Units (CFU) of the *Pediococcus* per 1 capsule serving and at least about 1 million CFU of the additional microorganism per 1 capsule serving. In another embodiment, the yield is about 150 billion CFU per gram of material. Other yields can also be used as desired.

In a further embodiment, the *Pediococcus* strain is one *or more of Pediococcus acidilactici, Pediococcus pentosaceus, Pediococcus damnosus, Pediococcus dextrinicus, or Pediococcus parvulus.*

In one preferred embodiment, *Pediococcus acidilactici* is used.

In one embodiment, the selected species of *Pediococcus* is combined with one or more further probiotics. The additional probiotic may be any microorganism that has a beneficial health effect in humans. Typically, the additional probiotic is one or more of: *Lactobacillus acidophilus, L. brevis, L. bulgaricus, L. casei, L. crispatus, L. curvatus, L. fermentum, L. gasseri, L. helveticus, L. johnsonii, L. paracasei, L. paraplantarum, L. pentosus, L. plantarum, L. reuteri, L. rhamnosus, L. salivarius, L. sakei, Lactococcus lactis, Leuconostoc lactis, Ln. pseudomesenteroides, Ln. mesenteroides, Bifidobacterium adolescentis, B. animalis, B. bifidum, B. breve, B. lactis, B. longum, B. infantis, Streptococcus thermophilus, Saccharomyces boulardii, Saccharomyces ceneviseae, Bacillus subtilis, B. coagulans* (frequently mislabeled as *Lactobacillus sporogenes*), *B. licheniformis, B. cereus, Enterococcus faecium, Escherichia coli* Nessle 1917, *Proprionibacterium acidipropionici, P. freudenreichii, P. jensenii,* and *P. thoenii.*

In one embodiment, none of the probiotic organisms in the composition have been or are propagated or grown in media containing casein or gluten.

In a further embodiment, the composition such as a dietary supplement is a dried powder, a tablet, a hydroxypropyl methylcellulose capsule, or a gelatin capsule. Exemplary methods for encapsulation of probiotics can be found, e.g., in US Patent Appl. 2007/0122397.

In a further embodiment, the composition such as a dietary supplement is administered subsequent to the administration of a digestive enzyme formulation, for example from immediately after administration of the digestive enzyme formulation to within the same day as administration of the digestive enzyme formulation or in parallel with the administration of a digestive enzyme formulation.

In one embodiment, the composition is supplied along with an ingestible support material for human consumption. Exemplary ingestible support materials include a cereal based product, rice cake, soy cake, food bar product, cold formed food bar product, custard, pudding, gelatin, rice milk, soy milk, mashed fruit product, candy, candy bar, and applesauce. Exemplary methods for encapsulation of probiotics can be found, e.g., in US Patent Appl 2007/0160589.

In one embodiment the product can be a kit or system wherein the compositions, capsules, etc., herein are contained in a pharmaceutically acceptable container and a written description, brochure, information sheet, catalog, or label explaining the product can reduce one or more symptoms of ASD and/or the product is free of casein and gluten and/or hypoallergenic. Further, the product can be marketed together with the written description, brochure, information sheet, catalog, or label explaining the product can reduce one or more symptoms of ASD and/or the product is free of casein and gluten. In an additional embodiment the product is marketed together with a written description, brochure, information sheet, catalog, or label explaining that the product is hypoallergenic.

Thus, in one aspect the current application can be directed to methods of reducing at least one symptom of an autism spectrum disorder (ASD) can comprise selecting an agent for the purpose of reducing at least one symptom of autism selected from twelve diagnostic criteria for autism according to the DSM-IV, 4th edition (2000), and administering a pharmaceutically acceptable composition can comprise a pharmaceutically effective amount of the agent to a person suspected of having autism for the purpose of reducing the at least one symptom, wherein the agent can comprise at least one probiotic *Pediococcus* strain and at least one second probiotic microbial organism in an amount and for a time adequate for statistically significant reduction of the at least one symptom, and wherein the second probiotic microbial organism can be at least one of a bacterium or a fungus.

In some embodiments, the agent can be delivered orally in a single serving capsule, the second probiotic microbial organism can be at least one of *Lactobacillus, Bifidobacterium* or *Saccharomyces,* and a dose or serving of the agent can comprise from about 1 million to 15 billion Colony Forming Units (CFU) of the *Pediococcus* and at least about 1 million CFU of the second probiotic microbial organism.

The dose or serving can comprise about 15 billion CFU of the *Pediococcus* per gram of pharmaceutically acceptable composition, and the *Pediococcus* strain can comprise at least one of *Pediococcus acidilactici, Pediococcus pentosaceus, Pediococcus damnosus, Pediococcus dextrinicus*, *or Pediococcus parvulus.* The second probiotic microbial organism can comprise at least one of *Lactobacillus acidophilus, Lactobacillus brevis, Lactobacillus bulgaricus, Lactobacillus casei, Lcectobacillus crispatus, Lactobacillus curvatus, Lactobacillus fermentum, Lactobacillus gasseri, Lactobacillus helveticus, Lactobacillus johnsonii, Lactobacillus paracasei, Lactobacillus paraplantarum*, *Lactobacillus pentosus*, *Lactobacillus plantarum, Lactobacillus reuteri, Lactobacillus rhamnosus, Lactobacillus salivarius, Lactobacillus sakei, Bifidobacterium adolescentis, Bifidobacterium animalis*, *Bifidobacterium bifidum, Bifidobacterium brave, Bifidobacterium lactis, Bifidobacterium longum, Bifidobacterium infantis, Saccharomyces boulardii,* and *Saccharomyces cereviseae.* The second probiotic microbial organism can also comprise at least one of *Lactococcus lactis, Leuconostoc lactis, Leuconostoc pseudomesenteroides*, *Leuconostoc mesenteroides, Streptococcus thermophilus*, *Bacillus subtilis, Bacillus coagulans, Bacillus licheniformis, Bacillus cereus*, *Enterococcus faecium, Escherichia coli* Nessle 1917, *Proprionibacterium acidipropionici, Proprionibacterium freudenreichii, Proprionibacterium jensenii,* and *Proprionibacterium thoenii.*

In still other embodiments, none of the probiotic microorganisms in the pharmaceutically acceptable composition have been propagated or grown in media containing casein and/or casein. The pharmaceutically acceptable composition can be configured and labeled as a dietary supplement, and the pharmaceutically acceptable composition can be a dried powder, a tablet, or within a gelatin capsule, which can be administered subsequent to administration of a digestive enzyme formulation to the person. The pharmaceutically acceptable composition can be provided within an ingestible support material for human consumption such as a cereal based product, rice cake, soy cake, food bar product, cold formed food bar product, custard, pudding, gelatin, rice milk, soy milk, mashed fruit product, candy, candy bar, and applesauce. The methods further can comprise reducing at least 3 or more symptoms of the ASD; reducing at least 6 or more symptoms of the ASD. The person receiving the administration can display at least six of the twelve symptoms in three categories of (1) impairments in social interaction, (2) impairments in communication, and (3) a restricted repertoire of activities and interests, and wherein the reducing of symptoms can comprise reducing at least one symptom in each of the three categories of symptoms. The method can also comprise reducing at least two symptoms from category (1), and at least one symptom from each of categories (2) and (3), or reducing at least two symptoms from each of categories (1), (2) and (3).

A digestive enzyme composition can be administered to the patient for a period of 1 day to 4 weeks prior to initiation of treatment with the pharmaceutically acceptable composition herein. The digestive enzyme composition can be administered to the patient for a period of 3 days to 1 week prior to initiation of treatment with the pharmaceutically acceptable composition herein. The pharmaceutically acceptable composition can be administered concomitantly with or subsequent to administration to the patient of an antibiotic agent or antifungal agent, which can be vancomycin. The pharmaceutically acceptable composition herein can be administered in conjunction with a gluten-free and/or casein-free diet, and can be administered in conjunction with administration to the patient of pharmaceutically effective amount of at least one of a casomorphin inhibitor, a gluteomorphin inhibitor, an enkephalin inhibitor, and/or an endorphin inhibitor, which inhibitor can be dipeptidylpeptidase IV or a dipeptidytpeptidase IV inducer.

The pharmaceutically acceptable composition can comprise about the following amounts of ingredients per serving or dose, where CFU means a Colony Forming Unit:
*Pediococcus acidilactici5* billion CFU
*Bifidobacterium breve.5* billion CFU
*Bifidobacterium infantis.5* billion CFU
*Lactobacillus paracasei.5* billion CFU
*Lactobacillus salivarius.5* billion CFU
*Bifidobacterium lactis* billion CFU
*Bifidobacterium longum* billion CFU
*Streptococcus thermophilus* billion CFU
*Lactobacillus bulgaricus* billion CFU
*Lactobacillus casei5* billion CFU
*Lactobacillus plantarum5* billion CFU
*Lactobacillus acidophilus* billion CFU
*Bifidobacterium bifidum5* billion CFU
*Lactobacillus rhamnosus* billion CFU

The pharmaceutically acceptable composition further can comprise at least one prebiotic agent that promotes the growth of probiotic microorganisms in the gastrointestinal tract. The prebiotic agent can comprise at least one of a fructooligosaccharide, galactooligosaccharide, lactulose, beta-glucan, inulin, pectin and resistant starch.

The autism spectrum disorder can be one or more of autism, childhood autism, Asperger's syndrome, Regressive autism, Rett syndrome, Childhood disintegrative disorder (CDD), Pathological demand avoidance syndrome (PDA).

In other aspects, the subject matter herein includes pharmaceutically acceptable compositions such as those described in the methods above as well as kits containing such compositions and for use in such methods. For example, such kits can comprise a vessel containing a composition as described herein and a label comprising instructions for pharmaceutical use of the composition to inhibit an autism spectrum disorder (ASD). The label can be an FDA approved label. In still other aspects, this application is directed to isolated and purified compositions as described herein for use in the manufacture of a medicament for inhibiting, preventing, or treating an autism spectrum disorder (ASD) in a human patient, as well as methods of manufacturing such medicaments, which can comprise combining a pharmaceutically effective amount of the composition and a pharmaceutically acceptable adjuvant, excipient, buffer or diluent.

These and other aspects, features and embodiments are set forth within this application, including the following Detailed Description. Unless expressly stated otherwise, all embodiments, aspects, features, etc., can be mixed and matched, combined and permuted in any desired manner.

### DETAILED DESCRIPTION

In one aspect, the compositions, capsules, methods, etc., herein comprise compositions for reducing the at least one and typically at least 3 or more symptoms of ASD, typically thereby effecting a statistically significant improvement in at least one of the symptoms included in the twelve diagnostic criteria for ASD according to the DSM-IV or Diagnosis and Statistical Manual for Mental Disorders, 4th edition, published by the American Psychiatric Association (Michael B. First, M.D., Editor, "Section 299.00 Autistic Disorder," Diagnostic and Statistical Manual--Text Revision, American Psychiatric Association, DSM IV-TR.TM., 2000).

These diagnostic criteria fall into three categories--impairments in social interaction, impairments in communication, and a restricted repertoire of activities and interests. A diagnosis of autism requires that a child display at least six of these twelve symptoms, with a minimum number in each category, namely, a total of six (or more) items from (1), (2), and (3), with at least two from (1), and one each from (2) and (3):
1. Qualitative impairment in social interaction, as manifested by at least two of the following: a. marked impairment in the use of multiple nonverbal behaviors such as eye-to-eye gaze, facial expression, body postures, and gestures to regulate social interaction, b. failure to develop peer relationships appropriate to developmental level, c. lack of spontaneous seeking to share enjoyment, interests, or achievements with other people (e.g., by a lack of showing, bringing, or pointing out objects of interest). d. lack of social or emotional reciprocity
2. Qualitative impairments in communication as manifested by at least one of the following: a. delay in, or total lack of, the development of spoken language (not accompanied by an attempt to compensate through alternative modes of communication such as gesture or mime), b. in individuals with adequate speech, marked impairment in the ability to initiate or sustain a conversation with others, c. stereotyped and repetitive use of language or idiosyncratic language, d. lack of varied, spontaneous make-believe play or social imitative play appropriate to developmental level
3. Restricted repetitive and stereotyped patterns of behavior, interests and activities, as manifested by at least one of the following: a. encompassing preoccupation with one or more stereotyped and restricted patterns of interest that is abnormal either in intensity or focus, b. apparently inflexible adherence to specific, nonfunctional routines or rituals, c. stereotyped and repetitive motor mannerisms (e.g., hand or finger flapping or twisting, or complex whole-body movements), d. persistent preoccupation with parts of objects.
   Individuals presenting with these diagnostic criteria may benefit from the present invention.

The reducing of symptoms can comprise reducing at least one or two symptoms in all of the three categories, for example at least two from (1), and one each from (2) and (3).

### Exemplary Autism Spectrum Disorders (ASD)

"Pervasive Developmental Disorder Not Otherwise Specified" (PDD-NOS) is a pervasive developmental disorder (PDD)/autism spectrum disorder (ASD) in which patients have some characteristics of disorders on the autistic spectrum, but do not fit the diagnostic criteria of any of the other autistic disorders thereon. While PDD-NOS shares similarities with classic autism, it tends to be milder. These patients have difficulties socializing, show repetitive behaviors, and are oversensitive to certain stimuli. In their interaction with others they might struggle to maintain eye contact, appear unemotional, or appear to be unable to speak. They may also have difficulty transitioning from one activity to another. PDD-NOS is referenced as the ICD-10 code F84.9. (International Classification of Diseases = ICD).

"Asperger's syndrome" is an autism spectrum disorder characterized by significant difficulties in social interaction, along with restricted and repetitive patterns of behavior and interests. It differs from other autism spectrum disorders by its relative preservation of linguistic and cognitive development.

"Regressive autism" is a form of autism in which the infant or child displays normal development up to a certain age (before age 3) at which s/he regresses or loses acquired abilities, e.g., speech, social interaction, etc. This is in contrast to infants who never hit their milestones and develop normally.

"Rett syndrome" is a genetic neurodevelopmental disorder of the grey matter of the brain that almost exclusively affects girls. It shares many features of autism. The clinical features include small hands and feet and a deceleration of the rate of head growth (including microcephaly in some). Repetitive hand movements, such as wringing and/or repeatedly putting hands into the mouth, are also noted. Gastrointestinal disorders are highly prevalent and up to 80% of patients have seizures.

"Childhood disintegrative disorder" (CDD), also called Heller's syndrome or disintegrative psychosis, is a rare condition characterized by late onset (>3 years of age) of developmental delays in language, social function, and motor skills. CDD has some similarity to autism, and is sometimes considered a low-functioning form of it, but an apparent period of fairly normal development is often noted before a regression in skills or a series of regressions in skills.

"Pathological demand avoidance syndrome" (PDA) is a diagnostic term more prevalent in the UK. In contrast to most individuals with autism spectrum disorders, individuals with PDA possess superficial social skills and seem to have a theory of mind. They often engage in manipulative, domineering behavior. The defining criteria are:
Passive early history in the first year, resisting ordinary demands and missing milestones
Continuing to resist demands, resorting to tantrums, distraction techniques Surface sociability, but apparent lack of sense of social identity, pride or shame Lability of mood and impulsivity
Comfortable in role play and pretending
Language delay, seemingly the result of passivity
Obsessive behavior
Neurological signs (awkwardness, similar to autism spectrum disorders).

Although not considered an ASD for all purposes, PDA is considered and ASD for purposes of this application.

### Probiotic Compositions

Probiotics include microorganisms that produce lactic acid as a major metabolic fermentation product. "Probiotics" is used in accord with its usual meaning, for example as selected, viable microbial dietary supplements that, when introduced in sufficient quantities, beneficially affect the human organism through their effects in the intestinal tract (Dimer C, Gibson GR (1998) An overview of probiotics, prebiotics and synbiotics in the functional food concept: perspectives and future strategies. Int Dairy J 8: 473-479; Zimmer CJ, Gibson GR (1998) An overview of probiotics, prebiotics and synbiotics in the functional food concept: perspectives and future strategies. Int Dairy J 8:473-479; Sanders ME (1998) Overview of functional foods: emphasis on probiotic bacteria. Int Dairy J 8: 341-347; Vaughan EE, Mollet B, de Vos WM (1999) Functionality of probiotics and intestinal lactobacilli: light in the intestinal tract tunnel. Curr Opin Biotechnol 10: 505-510; Zubillaga M, Weil R, Postaire E, Goldman C, Caro R, Boccio J (2001) Effect of probiotics and functional foods and their use in different diseases. Nutr Res 21: 569-579; Holzapfel WH, Schillinger U (2002) Introduction to preand probiotics. Food Res Int 35: 109-116. Also FAO/WHO has adopted the definition of probiotics as "Live microorganisms which when administered in adequate amounts confer a health benefit on the host". FAO/WHO (2002) Guidelines for the evaluation of probiotics in food. London, Ontario, Canada, April 30 and May 1, 2002. See: ftp://ftp.fao.org/es/esn/food/wgreport2.pdf.

These beneficial bacteria may be found for example in milk or in milk processing factories, living or decaying plants, and also in the intestines of man and animals (Holzapfel et al., 2001; Barros et al., 2001; O'Sullivan, 2001). Currently, possibly the best-studied probiotics are the lactic acid bacteria, particularly *Lactobacillus* spp. and *Bifidobacterium* spp. However, other organisms used as probiotics in humans include *Escherichia coli, Streptococcus* spp., *Enterococcus* spp., *Bacteroides* spp., *Bacillus* spp., *Propionibacterium* spp. and various fungi.

Probiotics have been examined for their effectiveness in the prevention and treatment of a diverse spectrum of gastrointestinal disorders such as antibiotic-associated diarrhea (including *Clostridium difficil*e-associated intestinal disease), infectious bacterial and viral diarrhea (including diarrhea caused by rotavirus, *Shigella, Salmonella,* enterotoxigenic *E. coli, Vibrio cholera* and human immunodeficiency virus/acquired immunodeficiency disorder, enteral feeding diarrhea, *Helicobacter pylori* gastroenteritis, sucrase maltase deficiency, inflammatory bowel disease, irritable bowel syndrome, small bowel bacterial overgrowth and lactose intolerance. Probiotics have been found to inhibit intestinal bacterial enzymes involved in the synthesis of colonic carcinogens. There are many mechanisms by which probiotics enhance intestinal health, including stimulation of immunity, competition for limited nutrients, inhibition of epithelial and mucosal adherence, inhibition of epithelial invasion and production of antimicrobial substances. Among other benefits, probiotic organisms are thought to restore and maintain immune system function and gastrointestinal barrier function (Salminen, 2001; Dunne et al., 2001; Parvez et al., 2006; Rolfe, 2000; Zareie, 2006).

*Lactobacillus* is a genus of Gram-positive facultative anaerobic bacteria. The genus *Lactobacillus* currently comprises over 100 species and encompasses a wide variety of organisms. They are common and usually benign. In humans they are present in the vagina and the gastrointestinal tract, where they are symbiotic and make up a small portion of the gut flora.

*Bifidobacterium* is a genus of Gram-positive anaerobic bacteria, currently comprised of 31 characterized species, 11 of which have been detected in human feces (Tannock, 1999). Bifidobacteria are irregular or branched rod-shaped bacteria that are commonly found in the intestines of humans and most animals and insects. They were first isolated and described over one hundred years ago and were quickly associated with a healthy gastrointestinal tract due to their prevalence in breast fed infants as compared with bottle fed infants (Tissier, 1906). While *B. infantis, B. brevi,* and *B. longum* are the largest group of bacteria in the intestine of infants, Bifidobacteria are said to be only the 3rd or 4th largest group in adults (and comprise only 3-6% of adult fecal flora). Bifidobacteria inhibit the growth of *Candida albicans, E. coli,* and other pathogenic bacteria. *B. infantis* has been shown to dramatically reduce the symptoms of irritable bowel syndrome (IBS) (Whorwell et al., 2006). *B. longum* is often the dominant species detected in humans and is a leading member of the probiotic bacteria, due to numerous studies that have provided a growing body of evidence for its potential health benefits. These include prevention of antibiotic-associated diarrhea (Black et al., 1991); cholesterol reduction (Dambekodi & Gilliland, 1998); alleviation of lactose intolerance symptoms (Jiang et al., 1996); immune stimulation (Takahashi et al., 1998); and cancer prevention (Reddy & Rivenson, 1993).

*Saccharomyces boulardii* is a transient yeast probiotic long used for various types of diarrhea. It is a hardy, acid-resistant, temperature tolerant microorganism that is not affected by antibiotics. *Saccharomyces boulardii* has been shown to suppress toxigenic *Clostridium difficile* overgrowth after vancomycin treatment in hamsters (Elmer & McFarland, 1987) and to prevent recurrences of *C*. *difficile*-associated colitis in humans (Surawicz et al., 1989). Recently, *S*. *boulardii* was shown to prevent the recurrence of Crohn's disease (Guslandi et al., 2000).

*Pediococcus* is a genus of lactic acid bacterium that is widely used in the food industry. *Pediococcus* can be described as "the only acidophilic, homofermentative, lactic acid bacteria that divide alternatively in two perpendicular directions to form tetrads" (Simpson and Taguchi, 1995). Phylogenetically, *Pediococcus* and *Lactobacillus* form a super-cluster that can be divided into two sub-clusters. All species of *Pediococcus* fall within the *Lactobacillus casei* - *Pediococcus* sub-cluster. Morphologically, *Pediococci* (cocci; 0.6-1.0 mm in diameter) and lactobacilli (rods) are distinct. Although eight species of *Pediococcus* were listed in the 1986 edition of the Bergey's manual, more recent information indicates that only five species currently belong to the genus: *Pediococcus acidilactici, Pediococcus damnosus, Pediococcus dextrinicus*, *Pediococcus parvulus,* and *Pediococcus pentosaceus*.

*P. pentosaceus* are acid tolerant and possess a fermentative metabolism with lactic acid as the major metabolic end product (Axelsson, 1998; Garvie, 1986). This organism is used as an acid producing starter culture in sausage fermentations, cucumber and green bean fermentations, soya milk fermentations, and silage (Simpson and Taguchi, 1995), and is a typical component of the adventitious or non-starter microflora of most cheese varieties during ripening (Beresford et al., 2001). Strains of *P. pentosaceus* have been reported to contain between three and five resident plasmids (Graham and McKay, 1985). Plasmid-linked traits include the ability to ferment raffinose, melibiose, and sucrose, as well as the production of antimicrobial peptides, which are also referred to as peptide bacteriocins (Daeschel and Klaenhammer, 1985; Gonzalez and Kunka, 1986).

Based on their primary structure, molecular mass, heat stability and molecular organization, bacteriocins produced by lactic acid bacteria can be subdivided into four classes (Klaenhammer 1993): class I, the lantibiotics (Jack *et* al. 1995, 1998; Sahl *et al*. 1995; Konings and Hilbers 1996); class II, the non-lantibiotic peptides (Nes *et al.* 1996), which are divided into the subgroups IIa or pediocin-like bacteriocins with strong antilisterial activity, IIb bacteriocins whose activity depends on the complementary action of two peptides, and IIc sec-dependent secreted bacteriocins; class III, large, heatlabile protein bacteriocins; and class IV, bacteriocins claimed to consist of an undefined mixture of protein(s), lipid(s) and carbohydrate(s).

The class IIa bacteriocins, often designated pediocin-like bacteriocins, are among the most important group of antimicrobial peptides produced by lactic acid bacteria (Drider et al., 2006). The peptide bacteriocins have properties that are of particular interest from a probiotic perspective. The bacteriocin PA-1/AcH, produced in high levels by some *P. acidilactici* strains (Marugg et al., 1992; Mora et al., 2000; Millette et al., 2007; Rodriguez et al., 2002) is active against a broad spectrum of Gram positive bacteria, including *Listeria monocytogenes, Staphylococcus aureus*, *Clostridium perfringens* and *Clostridium botulinum* (Bhunia *et al.,* 1988, 1991; Motlagh *et al.,* 1994; Okcreke & Montville, 1991; Pucci *et al.,* 1988). A strain of *Pediococcus acidilactici* denoted UVA1 was co-isolated from infant feces together with a *Bifidobacterium thermophilum* strain, and has strong antilisterial activity (Mathys et al., 2007).

*Pediococcus* has been used as a probiotic in the livestock and pet industries (US Patent Appl. 20060008511 and 20070020328). A mixture of *P. acidilactici* and *Saccharomyces boulardii* known as MitoMax^{®} (Imagilin Technology, Frederick, MD, USA) was shown to enhance the resistance of chickens against coccidiosis (Lee et al, 2007).

*P. pentosaceus* and *P. acidilactici* are generally recognized as safe for human consumption (GRAS) (Ishibashi & Yamazaki, 2001). Speelmans et al. (US Patent Appl. 2006/0165661; WO 2004/110466) described the use of pediocin-producing *Pediococci* for use against infections by multi-resistant pathogens in humans (US Patent application 20060165661). One commercially available probiotic mixture, known as Synbiotic 2000 MediFarm, Sweden, consists of a mixture of four probiotics from the *Lactobacillus* genera: *Pediococcus pentosaceus* 5-33:3, *Leuconostoc mesenteroides* 32-77:1, *Lactobacillus paracasei* subsp *paracasei* 19 and *Lactobacillus plantarum* 2362, along with four prebiotic substances: beta-glucan, inulin, pectin and digestion-resistant starch. This composition was reported to confer health benefits in individuals with arteriosclerosis, Crohn's disease and chronic liver disease (Kruszewska et al., 2002; Ljungh et al., 2002). However, this composition has not been described for reduction of symptoms of ASD.

Although not necessary to an understanding of the compositions, methods, etc., herein the beneficial effects of *Pediococcus* in reducing symptoms of autism may result not only from the production of bacteriocins, but also from the production of co-expressed cognate immunity proteins, which protect the producing organism from its own bacteriocin (Fimland et al., 2005). Without being bound by theory, these properties may allow these the *Pedioccoccus* compositions herein to confer a more normal neuro-gastrointestinal physiology in individuals with ASD compared to healthy persons.

The compositions described herein are useful in improving one or more of these 12 symptoms of ASD. Individuals that have been diagnosed with ASD according to the above criteria may benefit from the compositions included herein. It is not necessary that such individuals be diagnosed with a microbial or fungal infection, or an abnormal gastrointestinal flora, in order to benefit from this invention.

The compositions discussed herein may be administered, for example, as dietary supplements, food and beverage additives, food and beverage ingredients, and pharmaceutical agents. Any suitable administration route can be used, typically alimentary/orally.

The compositions discussed herein can include or be used in combination with compositions comprising digestive enzymes. For example, the compositions can be used in combination with a formulated combination of digestive enzymes known as EnzymAid™ (Kirkman Labs, Oregon) and a wide variety of other formulations. Typically, the digestive enzymes will be administered as a separate tablet or capsule or powder. A digestive enzyme formulation may if desired be given to a patient for a proscribed period of time prior to the initiation of treatment, for example for a period of 1-3 days to 1-4 weeks prior to initiation of treatment with the compositions described herein. Examples of digestive enzyme formulations that are suitable for use in the present invention include, but are not limited to, the products from ProThera Inc. and Klaire Labs, Inc., known as VitalZymes™ Complete; VitalZymes™ Forte; VitalZymes™ Chewables; and ScrenAid^{®}, and any of the ingredients therein (see: www.protheraine.com).

The compositions described herein may be used subsequent to treatment with antibiotic or antifungal agents, or concomitantly with such treatments. Since *Pediococcus* are intrinsically resistant to vancomycin (Riebel & Washington, 1990), the compositions, etc., herein can be used in autistic patients that are undergoing treatment with antibiotics such as vancomycin. However, the precedent, concomitant or subsequent use of vancomycin or any other antibiotic agent, or of any antifungal agent, is not a prerequisite.

The compositions discussed herein may be used in conjunction with a gluten-free and casein-free diet. In addition, the compositions, etc., herein may be used in conjunction with a casomorphin inhibitor, a gluteomorphin inhibitor, an enkephalin inhibitor, and/or an endorphin inhibitor. Thus, the methods and compositions of the present invention can be used in conjunction with any of the methods and compositions in Houston (US 6,447,772) and/or Wilkinson (US 6,251,391) including the SerenAid^{®} brand enzyme product from ProThera, Inc., www.protherainc.com. The pharmaceutically acceptable composition comprising the pharmaceutically effective amount of the agent can also administered in conjunction with administration to the patient of a pharmaceutically effective amount of dipeptidylpeptidase IV or a dipeptidylpeptidase IV inducer, which dipeptidylpeptidase IV can be the casomorphin inhibitor, a gluteomorphin inhibitor, an enkephalin inhibitor, and/or an endorphin inhibitor.

The compositions herein are preferably comprised of the following ingredients (% by relative CFU content).

| | |
|---|---|
| *Pediococcus acidilactici* | 1% to 99% |
| *Pediococcus pentosaceus* | 1% to 99% |
| *Pediococcus damnosus* | 1% to 99% |
| *Pediococcus dextrinicus* | 1% to 99% |
| *Pediococcus parvulus* | 1% to 99% |
| *Bifidobacterium lactis* | 1% to 99% |
| *Bifidobacterium animalis* | 0% to 99% |
| *Bifidobacterium adolescentis* | 0% to 98% |
| *Bifidobacterium bifidum* | 0% to 98% |
| *Bifidobacterium breve* | 0% to 98% |
| *Bifidobacterium infantis* | 0% to 98% |
| *Bifidobacterium loregum* | 0% to 98% |
| *Lactobacillus acidophilus* | 0% to 98% |
| *Lactobacillus brevis* | 0% to 98% |
| *Lactobacillus bulgaricus* | 0% to 98% |
| *Lactobacillus casei* | 0% to 98% |
| *Lactobacillus crispatus* | 0% to 98% |
| *Lactobacillus curvatus* | 0% to 98% |
| *Lactobacillus fermentum* | 0% to 98% |
| *Lactobacillus gasseri* | 0% to 98% |
| *Lactobacillus helveticus* | 0% to 98% |
| *Lactobacillus johnsonii* | 0% to 98% |
| *Lactobacillus paracasei* | 0% to 98% |
| *Lactobacillus paraplantarum* | 0% to 98% |
| *Lactobacillus pentosus* | 0% to 98% |
| *Lactobacillus plantarum* | 0% to 98% |
| *Lactobacillus reuteri* | 0% to 98% |
| *Lactobacillus rhamnosus* | 0% to 98% |
| *Lactobacillus sakei* | 0% to 98% |
| *Lactobacillus salivarius* | 0% to 98% |
| *Lactococcus lactis* | 0% to 98% |
| *Leuconostoc lactis* | 0% to 98% |
| *Leuconostoc pseudomesenteroides* | 0% to 98% |
| *Leuconostoc mesenteroides* | 0% to 98% |
| *Saccharomyces boulardii* | 0% to 98% |
| *Saccharomyces cereviseae* | 0% to 98% |
| *Stoeptococcus thermophilus* | 0% to 98% |
| *Bacillus subtilis* | 0% to 98% |
| *Bacillus coagulans* | 0% to 98% |
| *Bacillus licheniformis* | 0% to 98% |
| *Bacillus cereus* | 0% to 98% |
| *Enterococcusfaecium* | 0% to 98% |
| *Escherichia coli* Nessle 1917 | 0% to 98% |
| *Proprionibacterium acidipropionic* | 0% to 98% |
| *Proprionibacterium freudenreichii* | 0% to 98% |
| *Proprionibacteriumjensenii* | 0% to 98% |
| *Proprionibacterium thoenii* | 0% to 98% |
| *Enterococcus faecium* | 0% to 98% |

The above ingredients can also be present, for example, at ranges of more than 10%, 20%, 30%, 40%, 50%, 60%, 70%, 80%, 90%, or less than 90%, 80%, 70%, 60%, 50%, 40%, 30%, 20%, 10%.

In one embodiment, the compositions comprise about the following amounts of ingredients per 1 capsule serving size, where CFU means a Colony Forming Unit:

| | |
|---|---|
| *Pediococcus acidilactici* | 1.5 billion CFU (6.0%) |
| *Bifidobacterium breve* | 0.5 billion CFU (2.0%) |
| *Bifidobacterium infantis* | 0.5 billion CFU (2.0%) |
| *Lactobacillus paracasei* | 0.5 billion CFU (2.0%) |
| *Lactobacillus salivarius* | 0.5 billion CFU (2.0%) |
| *Bifidobacterium lactis* | 1.0 billion CFU (4.0%) |
| *Bifidobacterium longum* | 1.0 billion CFU (4.0%) |
| *Streptococcus thermophilus* | 1.0 billion CFU (4.0%) |
| *Lactobacillus bulgaricus* | 1.0 billion CFU (4.0%) |
| *Lactobacillus casei* | 2.5 billion CFU (10.0%) |
| *Lactobacillus plantarum* | 2.5 billion CFU (10.0%) |
| *Lactobacillus acidophilus* | 3.0 billion CFU (12.0%) |
| *Bifidobacterium bifidum* | 3.5 billion CFU (14.0%) |
| *Lactobacillus rhamnosus* | 6.0 billion CFU (24.0%) |

It will be understood that a variety of different mixtures of *Lactobacillus Bifidobacterium, Streptococcus, Lactococcus,* and other probiotic organisms can be combined with *Pediococcus* in various % compositions and doses that produce efficacious results; the invention is not limited to the exact formulation described above.

In addition, the compositions, methods, etc., herein can be formulated, made or used to include prebiotic agents that promote the growth of probiotic organisms in the gastrointestinal tract. Suitable prebiotic agents include, but are not limited to, fructooligosaccharides, galactooligosaccharides, lactulose, beta-glucan, inulin, pectin and resistant starch (see, e.g., MacFarlane et al., 2006; Paul et al., US 6,241,983).

All terms used herein are used in accordance with their ordinary meanings unless the context or definition clearly indicates otherwise. Also unless expressly indicated otherwise, in the specification the use of "or" includes "and" and vice-versa. Non-limiting terms are not to be construed as limiting unless expressly stated, or the context clearly indicates, otherwise (for example, "including," "having," and "comprising" typically indicate "including without limitation"). Singular forms, including in the claims, such as "a," "an," and "the" include the plural reference unless expressly stated, or the context clearly indicates, otherwise.

The scope of the present devices, systems and methods, etc., includes both means plus function and step plus function concepts. However, the claims are not to be interpreted as indicating a "means plus function" relationship unless the word "means" is specifically recited in a claim, and are to be interpreted as indicating a "means plus function" relationship where the word "means" is specifically recited in a claim. Similarly, the claims are not to be interpreted as indicating a "step plus function" relationship unless the word "step" is specifically recited in a claim, and are to be interpreted as indicating a "step plus function" relationship where the word "step" is specifically recited in a claim.

From the foregoing, it will be appreciated that, although specific embodiments have been discussed herein for purposes of illustration, various modifications may be made without deviating from the spirit and scope of the discussion herein. Accordingly, the systems and methods, etc., include such modifications as well as all permutations and combinations of the subject matter set forth herein and are not limited except as by the appended claims or other claim having adequate support in the discussion and figures herein.

The invention is further described in the following paragraphs:
1. A method of reducing at least one symptom of an autism spectrum disorder (ASD) comprising selecting an agent for the purpose of reducing at least one symptom of autism selected from twelve diagnostic criteria for autism according to the DSM-IV, 4th edition (2000), and administering a pharmaceutically acceptable composition comprising a pharmaceutically effective amount of the agent to a person suspected of having autism for the purpose of reducing the at least one symptom, wherein the agent comprises at least one probiotic *Pediococcus* strain and at least one second probiotic microbial organism in an amount and for a time adequate for statistically significant reduction of the at least one symptom, and wherein the second probiotic microbial organism is at least one of a bacterium or a fungus.
2. The method of paragraph 1 wherein the second probiotic microbial organism is at least one of *Lactobacillus, Bifidobacterium* or *Saccharomyces.*
3. The method of paragraph 1 or 2 wherein the agent is delivered orally in a single serving capsule.
4. The method of any one of paragraphs 1 to 3 wherein a dose or serving of the agent comprises from about 1 million to 150 billion Colony Forming Units (CFU) of the *Pediococcus* and at least about 1 million CFU of the second probiotic microbial organism.
5. The method of paragraph 4 wherein the dose or serving comprises about 150 billion CFU of the *Pediococcus* per gram of pharmaceutically acceptable composition.
6. The method of any one of paragraphs 1 to 5 wherein the *Pediococcus* strain comprises at least one of *Pediococcus acidilactici, Pediococcus pentosaceus, Pediococcus damnosus, Pediococcus dextrinicus, or Pediococcus parvulus.*
7. The method of any one of paragraphs 1 to 6 wherein the *Pediococcus* strain comprises *Pediococcus acidilactici.*
8. The method of any one of paragraphs 1 to 7 wherein the second probiotic microbial organism comprises at least one of *Lactobacillus acidophilus, Lactobacillus brevis, Lactobacillus bulgaricus, Lactobacillus casei, Lactobacillus crispatus, Lactobacillus curvatus, Lactobacillus fermentum, Lactobacillus gasseri, Lactobacillus helveticus, Lactobacillus johnsonii, Lactobacillus paracasei, Lactobacillus paraplantarum, Lactobacillus pentosus, Lactobacillus plantarum, Lactobacillus reuteri, Lactobacillus rhamnosus, Lactobacillus salivarius, Lactobacillus sakei, Bifidobacterium adolescentis, Bifidobacterium animalis, Bifidobacterium bifidum, Bifidobacterium breve, Bifidobacterium lactis, Bifidobacterium longum, Bifidobacterium infantis, Saccharomyces boulardii,* and *Saccharomyces cereviseae.*
9. The method of paragraph 1 or 3 to 7 wherein the second probiotic microbial organism comprises at least one of *Lactococcus lactis, Leuconostoc lactis, Leuconostoc pseudomesenteroides, Leuconostoc mesenteroides, Streptococcus thermophilus, Bacillus subtilis, Bacillus coagulans, Bacillus licheniformis, Bacillus cereus, Enterococcus faecium, Escherichia coli* Nessle 1917, *Proprionibacterium acidipropionici, Proprionibacterium freudenreichii, Proprionibacterium jensenii,* and *Proprionibacterium thoenii.*
10. The method of any one of paragraphs 1 to 9 wherein none of the probiotic microorganisms in the pharmaceutically acceptable composition have been propagated or grown inmedia containing casein.
11. The method of any one of paragraphs 1 to 9 wherein none of the probiotic microorganisms in the pharmaceutically acceptable composition have been propagated or grown in media containing gluten.
12. The method of any one of paragraphs 1 to 9 wherein none of the probiotic microorganisms in the pharmaceutically acceptable composition have been propagated or grown in media containing casein or gluten.
13. The method of any one of paragraphs 1 to 12 wherein the pharmaceutically acceptable composition is configured and labeled as a dietary supplement.
14. The method of any one of paragraphs 1 to 13 wherein the pharmaceutically acceptable composition is a dried powder, a tablet, or within a gelatin capsule.
15. The method of any one of paragraphs 1 to 14 wherein the pharmaceutically acceptable composition is administered subsequent to administration of a digestive enzyme formulation to the person.
16. The method of any one of paragraphs 1 to 15 wherein the pharmaceutically acceptable composition is provided within an ingestible support material for human consumption.
17. The method of paragraph 14 wherein the ingestible support material is one of a cereal based product, rice cake, soy cake, food bar product, cold formed food bar product, custard, pudding, gelatin, rice milk, soy milk, mashed fruit product, candy, candy bar, and applesauce.
18. The method of any one of paragraphs 1 to 17 wherein the method further comprises reducing at least 3 or more symptoms of the ASD.
19. The method of any one of paragraphs 1 to 18 wherein the method further comprises reducing at least 6 or more symptoms of the ASD.
20. The method of any one of paragraphs **1** to 19 wherein the person receiving the administration displays at least six of the twelve symptoms in three categories of **(1)** impairments in social interaction, (2) impairments in communication, and (3) a restricted repertoire of activities and interests, and wherein the reducing of symptoms comprises reducing at least one symptom in each of the three categories of symptoms.
21. The method of paragraph 20 wherein the method further comprises reducing at least two symptoms from category (1), and at least one symptom from each of categories (2) and (3).
22. The method of paragraph 20 wherein the method further comprises reducing at least two symptoms from each of categories (1), (2) and(3).
23. The method of any one of paragraphs 1 to 22 wherein a digestive enzyme composition is administered to the patient for a period of 1 day to 4 weeks prior to initiation of treatment with the pharmaceutically acceptable composition comprising the pharmaceutically effective amount of the agent.
24. The method of paragraph 23 wherein the digestive enzyme composition 1s administered to the patient for a period of 3 days to **1** week prior to initiation of treatment with the pharmaceutically acceptable composition comprising the pharmaceutically effective amount of the agent.
25. The method of any one of paragraphs **1** to 24 wherein the pharmaceutically acceptable composition comprising the pharmaceutically effective amount of the agent 1s administered subsequent to administration to the patient of an antibiotic agent or antifungal agent.
26. The method of paragraph 25 wherein the antibiotic agent or antifungal agent 1s vancomycin.
27. The method of any one of paragraphs 1 to 24 wherein the pharmaceutically acceptable composition comprising the pharmaceutically effective amount of the agent is administered concomitantly with administration to the patient of an antibiotic agent or antifungal agent.
28. The method of paragraph 27 wherein the antibiotic agent or antifungal agent 1s vancomycin.
29. The method of any one of paragraphs 1 to 28 wherein the pharmaceutically acceptable composition comprising the pharmaceutically effective amount of the agent is administered in conjunction with a gluten-free and casein-free diet.
30. The method of any one of paragraphs 1 to 29 wherein the pharmaceutically acceptable composition comprising the pharmaceutically effective amount of the agent is administered in conjunction with administration to the patient of pharmaceutically effective amount of at least one of a casomorphin inhibitor, a gluteomorphin inhibitor, an enkephalin inhibitor, and/or an endorphin inhibitor.
31. The method of paragraph 30 wherein the pharmaceutically acceptable composition comprising the pharmaceutically effective amount of the agent is administered in conjunction with administration to the patient of a pharmaceutically effective amount of dipeptidylpeptidase IV or a dipeptidylpeptidase IV inducer.
32. The method of any one of paragraphs 1 to 31 wherein the pharmaceutically acceptable composition comprises about the following amounts of ingredients per serving or dose, where CFU means a Colony Forming Unit:

| | |
|---|---|
| *Pediococcus acidilactici* | 1.5 billion CFU |
| *Bifidobacterium breve* | 0.5 billion CFU |
| *Bifidobacterium infantis* | 0.5 billion CFU |
| *Lactobacillus paracasei* | 0.5 billion CFU |
| *Lactobacillus salivarius* | 0.5 billion CFU |
| *Bifidobacterium lactis* | 1.0 billion CFU |
| *Bifidobacterium longum* | 1.0 billion CFU |
| *Streptococcus thermophilus* | 1.0 billion CFU |
| *Lactobacillus bulgaricus* | 1.0 billion CFU |
| *Lactobacillus casei* | 2.5 billion CFU |
| *Lactobacillus plantarum* | 2.5 billion CFU |
| *Lactobacillus acidophilus* | 3.0 billion CFU |
| *Bifidobacterium bifidum* | 3.5 billion CFU |
| *Lactobacillus rhamnosus* | 6.0 billion CFU |

33. The method of any one of paragraphs 1 to 32 wherein the pharmaceutically acceptable composition further comprises at least one prebiotic agent that promotes the growth of probiotic microorganisms in the gastrointestinal tract.
34. The method of paragraph 33 wherein the prebiotic agent comprises at least one of a fructooligosaccharide, galactooligosaccharide, lactulose, beta-glucan, inulin, pectin and resistant starch.
35. The method of any one of paragraphs 1 to 34 wherein the autism spectrum disorder is autism.
36. The method of any one of paragraphs 1 to 34 wherein the autism spectrum disorder is Asperger's syndrome.
37. The method of any one of paragraphs 1 to 34 wherein the autism spectrum disorder is Regressive autism.
38. The method of any one of paragraphs 1 to 34 wherein the autism spectrum disorder is Rett syndrome.
39. The method of any one of paragraphs 1 to 34 wherein the autism spectrum disorder is Childhood disintegrative disorder(CDD).
40. The method of any one of paragraphs 1 to 34 wherein the autism spectrum disorder is Pathological demand avoidance syndrome (PDA).
41. A pharmaceutically acceptable composition configured to at least one symptom of autism selected from twelve diagnostic criteria for autism according to the DSM-IV, 4th edition (2000), the composition comprising at least one probiotic *Pediococcus* strain and at least one second probiotic microbial organism in an amount adequate to cause statistically significant reduction of at least one symptom of autism selected from twelve diagnostic criteria for autism according to the DSM-IV, 4th edition (2000), and wherein the second probiotic microbial organism is at least one of a bacterium or a fungus.
*42.* The composition of paragraph 41 wherein the second probiotic microbial organism is at least one of *Lactobacillus, Bifidobacterium* or *Saccharomyces.*
43. The composition of any one of paragraphs 41 to 42 wherein the pharmaceutically acceptable composition is located in a pharmaceutically acceptable container and is labeled as a dietary supplement.
44. The composition of any one of paragraphs 41 to 43 wherein a dose or serving of the agent comprises from about 1 million to 150 billion Colony Forming Units (CFU) of the *Pediococcus* and at least about 1 million CFU of the second probiotic microbial organism.
45. The composition of any one of paragraphs 41 to 44 wherein a dose or servmg comprises about 150 billion CFU of the *Pediococcus* per gram of pharmaceutically acceptable composition.
*46.* The composition of any one of paragraphs 41 to 45 wherein the *Pediococcus* strain comprises at least one of *Pediococcus acidilactici, Pediococcus pentosaceus, Pediococcus damnosus, Pediococcus dextrinicus, or Pediococcus parvulus.*
*47.* The composition of any one of paragraphs 41 to 46 wherein the *Pediococcus* strain comprises *Pediococcus acidilactici.*
*48.* The composition of any one of paragraphs 41 to 47 wherein the second probiotic microbial organism comprises at least one of *Lactobacillus acidophilus, Lactobacillus brevis, Lactobacillus bulgaricus, Lactobacillus casei, Lactobacillus crispatus, Lactobacillus curvatus, Lactobacillus fermentum, Lactobacillus gasseri, Lactobacillus helveticus, Lactobacillus johnsonii, Lactobacillus paracasei, Lactobacillus paraplantarum, Lactobacillus pentosus, Lactobacillus plantarum, Lactobacillus reuteri, Lactobacillus rhamnosus, Lactobacillus salivarius, Lactobacillus sakei, Bifidobacterium adolescentis, Bifidobacterium animalis, Bifidobacterium bifidum, Bifidobacterium breve, Bifidobacterium lactis, Bifidobacterium longum, Bifidobacterium infantis, Saccharomyces boulardii,* and *Saccharomyces cereviseae.*
*49.* The composition of any one of paragraphs 41 to 48 wherein the second probiotic microbial organism comprises at least one of *Lactococcus lactis, Leuconostoc lactis, Leuconostoc pseudomesenteroides, Leuconostoc mesenteroides, Streptococcus thermophilus, Bacillus subtilis, Bacillus coagulans, Bacillus licheniformis, Bacillus cereus, Enterococcus faecium, Escherichia coli* Nessle 1917, *Proprionibacterium acidipropionici, Proprionibacterium freudenreichii, Proprionibacterium jensenii,* and *Proprionibacterium thoenii.*
50. The composition of any one of paragraphs 41 to 49 wherein none of the probiotic microorganisms in the pharmaceutically acceptable composition have been propagated or grown inmedia containing casein.
51. The composition of any one of paragraphs 41 to 50 none of the probiotic microorganisms in the pharmaceutically acceptable composition have been propagated or grown in media containing gluten.
52. The composition of any one of paragraphs 41 to 51 wherein none of the probiotic microorganisms in the pharmaceutically acceptable composition have been propagated or grown in media containing casein or gluten.
53. The composition of any one of paragraphs 41 to 52 wherein the pharmaceutically acceptable composition is a dried powder, a tablet, or within a gelatin capsule.
54. The composition of any one of paragraphs 41 to 53 wherein the pharmaceutically acceptable composition is provided within an ingestible support material for human consumption.
55. The composition paragraph 54 wherein the ingestible support material is one of a cereal based product, rice cake, soy cake, food bar product, cold formed food bar product, custard, pudding, gelatin, rice milk, soy milk, mashed fruit product, candy, candy bar, and applesauce.
56. The composition of any one of paragraphs 41 to 55 wherein the composition is further configured to reduce at least 3 or more symptoms of the ASD.
57. The composition of any one of paragraphs 41 to 56 wherein the composition is further configured to reduce at least 6 or more symptoms of the ASD.
58. The composition of any one of paragraphs 41 to 57 wherein the composition is further configured to reduce at least one symptom in each of the three categories of symptoms.
59. The method of paragraph 58 wherein the composition is further configured to reduce at least two symptoms in each of the three categories of symptoms.
60. The composition of any one of paragraphs 41 to 59 wherein the composition further comprises at least one of an antibiotic agent or antifungal agent.
61. The composition of any one of paragraphs 41 to 60 wherein the composition further comprises an antibiotic agent and an antifungal agent
62. The composition of paragraph 61 wherein the antibiotic agent or antifungal agent is vancomycin.
63. The composition of any one of paragraphs 41 to 62 wherein the pharmaceutically acceptable composition further comprises at least one of a casomorphin inhibitor, a gluteomorphin inhibitor, an enkephalin inhibitor, and/or an endorphin inhibitor.
64. The composition of any one of paragraphs 41 to 62 wherein the pharmaceutically acceptable composition further comprises a pharmaceutically effective amount of dipeptidylpeptidase IV or a dipeptidylpeptidase IV inducer.
65. The composition of any one of paragraphs 41 to 64 wherein the pharmaceutically acceptable composition comprises about the following amounts of ingredients per serving or dose, where CFU means a Colony Forming Unit:

| | |
|---|---|
| *Pediococcus acidilactici* | 1.5 billion CFU |
| *Bifidobacterium breve* | 0.5 billion CFU |
| *Bifidobacterium infantis* | 0.5 billion CFU |
| *Lactobacillus paracasei* | 0.5 billion CFU |
| *Lactobacillus salivarius* | 0.5 billion CFU |
| *Bifidobacterium lactis* | 1.0 billion CFU |
| *Bifidobacterium longum* | 1.0 billion CFU |
| *Streptococcus thermophilus* | 1.0 billion CFU |
| *Lactobacillus bulgaricus* | 1.0 billion CFU |
| *Lactobacillus casei* | 2.5 billion CFU |
| *Lactobacillus plantarum* | 2.5 billion CFU |
| *Lactobacillus acidophilus* | 3.0 billion CFU |
| *Bifidobacterium bifidum* | 3.5 billion CFU |
| *Lactobacillus rhamnosus* | 6.0 billion CFU |

66. The composition of any one of paragraphs 41 to 65 wherein the pharmaceutically acceptable composition further comprises at least one prebiotic agent that promotes the growth of probiotic microorganisms in the gastrointestinal tract.
67. The method of paragraph 66 wherein the prebiotic agent comprises at least one of a fructooligosaccharide, galactooligosaccharide, lactulose, beta-glucan, inulin, pectin and resistant starch.
68. A kit comprising a vessel containing a composition according to any one of paragraphs 41 to 67 and a label comprising instructions for pharmaceutical use of the composition to inhibit an autism spectrum disorder (ASD).
69. The kit of paragraph 68 wherein the label is an FDA approved label.
70. An isolated and purified composition according to any one of paragraphs 41 to 67 for use in the manufacture of a medicament for inhibiting, preventing, or treating an autism spectrum disorder (ASD) ina human patient.
71. A method of manufacturing a medicament able to reduce symptoms associated inhibiting, preventing, or treating an autism spectrum disorder (ASD) in a human patient, comprising combining a pharmaceutically effective amount of a composition according to any one of paragraphs 41 to 67, and a pharmaceutically acceptable adjuvant, excipient, buffer or diluent.

## Claims

1. A pharmaceutically acceptable composition for use in a method of inhibiting, preventing, or treating an autism spectrum disorder (ASD) in a human patient, said composition comprising (i) at least one probiotic Pediococcus strain and (ii) at least one second probiotic microbial organism in an amount adequate to cause statistically significant reduction of at least one symptom of autism selected from twelve diagnostic criteria for autism according to the DSM-IV, 4th edition (2000), wherein the autism spectrum disorder is autism, Asperger's syndrome, Regressive autism, Rett syndrome, Childhood disintegrative disorder (CDD) or Pathological demand avoidance syndrome (PDA) and wherein a dose or serving of the probiotic agent comprises from about 1 million to 150 billion Colony Forming Units (CFU) of the Pediococcus and at least about 1 million CFU of the second probiotic microbial organism.

2. The composition for the use of claim 1 wherein the second probiotic microbial organism is at least one of Lactobacillus, Bifidobacterium or Saccharomyces.

3. The composition for the use of claim 1 or 2 , wherein the pharmaceutically acceptable composition is located in a pharmaceutically acceptable container and is labeled as a dietary supplement.

4. The composition for the use of any one of claims 1 to 3, wherein a dose or serving comprises about 150 billion CFU of the Pediococcus per gram of pharmaceutically acceptable composition.

5. The composition for the use of any one of claims 1 to 4, wherein the Pediococcus strain comprises at least one of Pediococcus acidilactici, Pediococcus pentosaceus, Pediococcus damnosus, Pediococcus dextrinicus, or Pediococcus parvulus.

6. The composition for the use of any one of claims 1 to 5, wherein the second probiotic microbial organism comprises at least one of Lactobacillus acidophilus, Lactobacillus brevis, Lactobacillus bulgaricus, Lactobacillus casei, Lactobacillus crispatus, Lactobacillus curvatus, Lactobacillus fermentum, Lactobacillus gasseri, Lactobacillus helveticus, Lactobacillus johnsonii, Lactobacillus paracasei, Lactobacillus paraplantarum, Lactobacillus pentosus, Lactobacillus plantarum, Lactobacillus reuteri, Lactobacillus rhamnosus, Lactobacillus salivarius, Lactobacillus sakei, Bifidobacterium adolescentis, Bifidobacterium animalis, Bifidobacterium bifidum, Bifidobacterium breve, Bifidobacterium lactis, Bifidobacterium longum, Bifidobacterium infantis, Saccharomyces boulardii, and Saccharomyces cereviseae.

7. The composition for the use of any one of claims 1 to 6, wherein the second probiotic microbial organism comprises at least one of Lactococcus lactis, Leuconostoc lactis, Leuconostoc pseudomesenteroides, Leuconostoc mesenteroides, Streptococcus thermophilus, Bacillus subtilis, Bacillus coagulans, Bacillus licheniformis, Bacillus cereus, Enterococcus faecium, Escherichia coli Nessle 1917, Proprionibacterium acidipropionici, Proprionibacterium freudenreichii, Proprionibacterium jensenii, and Proprionibacterium thoenii.

8. The composition for the use of any one of claims 1 to 7, wherein none of the probiotic microorganisms in the pharmaceutically acceptable composition have been propagated or grown in media containing casein and/or gluten.

9. The composition for the use of any one of claims 1 to 8, wherein the pharmaceutically acceptable composition is a dried powder, a tablet, or within a gelatin capsule.

10. The composition for the use of any one of claims 1 to 9, wherein the pharmaceutically acceptable composition is provided within an ingestible support material for human consumption.

11. The composition for the use of claim 10, wherein the ingestible support material is one of a cereal based product, rice cake, soy cake, food bar product, cold formed food bar product, custard, pudding, gelatin, rice milk, soy milk, mashed fruit product, candy, candy bar, and applesauce.

12. The composition for the use of any one of claims 1 to 11, wherein the composition further comprises at least one of an antibiotic agent or antifungal agent, preferably wherein the composition further comprises an antibiotic agent and an antifungal agent.

13. The composition for the use of claim 12, wherein the antibiotic agent or antifungal agent is vancomycin.

14. The composition for the use of claim 12 or 13, wherein a pharmaceutically effective amount of the agent is administered subsequent or concomitantly with the administration of the antibiotic or antifungal agent to the patient.

15. The composition for the use of any one of claims 1 to 14, wherein the pharmaceutically acceptable composition further comprises at least one of a casomorphin inhibitor, a gluteomorphin inhibitor, an enkephalin inhibitor, and/or an endorphin inhibitor.

16. The composition for the use of any one of claims 1 to 15, wherein the pharmaceutically acceptable composition further comprises a pharmaceutically effective amount of dipeptidylpeptidase IV or a dipeptidylpeptidase IV inducer.

17. The composition for the use of any one of claims 1 to 16, wherein the pharmaceutically acceptable composition comprises about the following amounts of ingredients per serving or dose, where CFU means a Colony Forming Unit:
Pediococcus acidilactici 1.5 billion CFU
Bifidobacterium breve 0.5 billion CFU
Bifidobacterium infantis 0.5 billion CFU
Lactobacillus paracasei 0.5 billion CFU
Lactobacillus salivarius 0.5 billion CFU
Bifidobacterium lactis 1.0 billion CFU
Bifidobacterium longum 1.0 billion CFU
Streptococcus thermophilus 1.0 billion CFU
Lactobacillus bulgaricus 1.0 billion CFU
Lactobacillus casei 2.5 billion CFU
Lactobacillus plantarum 2.5 billion CFU
Lactobacillus acidophilus 3.0 billion CFU
Bifidobacterium bifidum 3.5 billion CFU
Lactobacillus rhamnosus 6.0 billion CFU

18. The composition for the use of any one of claims 1 to 17, wherein the pharmaceutically acceptable composition further comprises at least one prebiotic agent that promotes the growth of probiotic microorganisms in the gastrointestinal tract.

19. The composition for the use of claim 18, wherein the prebiotic agent comprises at least one of a fructooligosaccharide, galactooligosaccharide, lactulose, beta-glucan, inulin, pectin and resistant starch.

20. The composition for the use according to any of claims 1 to 19, wherein a digestive enzyme composition is administered to the patient for a period of 1 day to 4 weeks, preferably 3 days to 1 week, prior to initiation of treatment with the pharmaceutically acceptable composition.

21. The composition for the use according to any of claims 1 to 20, wherein the pharmaceutically acceptable composition is administered in conjunction with a gluten-free and casein-free diet.
